# EUROPEAN PATENT SPECIFICATION

(11) **EP 4 554 513 B1**
(45) Date of publication and mention of the grant of the patent: **18.11.2026**
(21) Application number: 23748321.9
(22) Date of filing: 11.07.2023
(51) Int. Cl.: A61F 2/16

(54) **HYDRAULIC DELIVERY OF SURGICAL IMPLANTS**
HYDRAULISCHE ABGABE VON CHIRURGISCHEN IMPLANTATEN
ADMINISTRATION HYDRAULIQUE D'IMPLANTS CHIRURGICAUX

(30) Priority: 12.07.2022 US 202263368173 P
(43) Date of publication of application: 21.05.2025
(73) Proprietor: Alcon Inc., 1701 Fribourg (CH)
(72) Inventor: TABER, Todd, Fort Worth, Texas 76134 (US); LEE, IV, Jestwin Edwin, Fort Worth, Texas 76134 (US); JENSEN, Kathryn, Fort Worth, Texas 76134 (US); MAGADUM, Pradeep, Fort Worth, Texas 76134 (US); YADAV, Saumya Dilip, Fort Worth, Texas 76134 (US)
(74) Representative: Murgitroyd & Company
(86) International application number: PCT/IB2023/057106
(87) International publication number: WO 2024/013661

(56) References cited:
- WO-A1-2020/128762
- WO-A1-2021/154203
- WO-A1-2022/101690
- WO-A1-2022/118249

## Description

### TECHNICAL FIELD

The invention set forth in the appended claims relates generally to eye surgery. More particularly, but without limitation, the claimed subject matter relates to systems, apparatuses, and methods for inserting an implant into an eye.

### BACKGROUND

The human eye can suffer a number of maladies causing mild deterioration to complete loss of vision. While contact lenses and eyeglasses can compensate for some ailments, ophthalmic surgery may be required for others. In some instances, implants may be beneficial or desirable. For example, an intraocular lens may replace a clouded natural lens within an eye to improve vision.

While the benefits of intraocular lenses and other implants are known, improvements to delivery systems, components, and processes continue to improve outcomes and benefit patients.

Reference is made to the documents WO 2021/154203 A1 and WO 2020/128762 A1 which have been cited as exemplary of the state of the art. WO 2021/154203 A1 describes a delivery device for inserting an intraocular lens into an eye which uses an inner plunger and an outer plunger cavity interacting with a fluid-containing cartridge. When the delivery mechanism is operated, fluid from the cartridge is displaced to assist in pushing or lubricating the implant.

### BRIEF SUMMARY

The scope of the invention is in accordance with the appended claims.

Illustrative embodiments are also provided to enable a person skilled in the art to make and use the claimed subject matter.

For example, some embodiments may comprise or consist essentially of an apparatus for delivering an implant, such as an intraocular lens, using hydraulic pressure or fluid flow. The apparatus may be combined with a disposable hydraulic cartridge, such as a vial of working fluid, to provide a fully disposable system for storing, advancing, and delivering an implant.

In more particular examples, the apparatus may comprise a rigid plunger for advancing an implant to a sealed position in a first phase, and a bore through the rigid plunger that allows a working fluid to advance the implant into the eye via hydraulic pressure in a second phase.

For example, a hollow rigid plunger can be used to first advance an intraocular lens with haptics in a straightened configuration to a point that a seal is created about the intraocular lens within a delivery
lumen. The lens may then be hydraulically advanced to delivery by passing a working fluid through the hollow bore of the plunger. The apparatus may additionally comprise a plunger stop, which may be configured to stop advancement of the implant interface or the plunger.

In yet more particular embodiments, the apparatus may have a nozzle, a plunger, a plunger rod, and a chamber configured to receive a cartridge between the plunger and the plunger rod. To maintain a compact configuration, the plunger rod may be placed in a forward position for shipping and storage. For operation, the plunger rod may be placed in an aft position to allow the cartridge to be inserted into the chamber between the plunger and the plunger rod. The plunger rod may be advanced toward the cartridge to couple the cartridge to the plunger. The plunger rod, the cartridge, and the plunger may be advanced further to advance the implant to a sealed position in the nozzle. Further advancement of the plunger rod can drive a working fluid from the cartridge and through a bore in the plunger to eject the implant from the nozzle.

More generally, a system for using a cartridge for eye surgery may comprise a nozzle, an implant bay coupled to the nozzle, an implant disposed in the implant bay, and an actuator. The actuator may comprise a plunger and a chamber configured to receive the cartridge. The actuator may be configured to fluidly couple the cartridge to the implant bay. In a first delivery phase, the actuator may move the plunger to advance the implant into the nozzle. In a second delivery phase, the actuator may move a working fluid from the cartridge through the plunger to advance the implant through the nozzle. In more particular embodiments, the actuator may further comprise a plunger rod and a bore through the plunger. The plunger rod may be configured to advance the plunger and to move the working fluid from the cartridge through the bore to advance the implant through the nozzle. Some embodiments of the plunger may comprise a coupling configured to fluidly couple the cartridge to the implant bay. Additionally, or alternatively, some embodiments of the system may comprise a lead nut, which may be threaded onto a threaded portion of the plunger rod. The plunger rod may be configured to be rotated through the lead nut to move the working fluid and may be configured to maintain a relative position between the plunger rod and the cartridge as the implant is advanced from the implant bay to the nozzle. In yet more particular embodiments, the cartridge may comprise a cartridge seal, and the plunger rod may be configured to advance the cartridge seal to move the working fluid. For example, the plunger rod may be configured to be rotated through a lead nut to advance the cartridge seal.

In other examples, a system for using a hydraulic cartridge for eye surgery may comprise a nozzle, an implant bay coupled to the nozzle, an implant disposed in the implant bay, a housing coupled to the implant bay, a plunger disposed within the housing, and a plunger rod at least partially disposed within the housing. The system may additionally comprise a bore through the plunger, which can be fluidly coupled to the implant bay. A chamber may be configured to receive the hydraulic cartridge between the plunger and the plunger rod. The plunger rod may be configured to move the hydraulic cartridge and the plunger within the housing to advance the implant from the implant bay to the nozzle in a first delivery phase. The plunger rod may be operable to drive a working fluid from the hydraulic cartridge through the bore to advance the implant through the nozzle in a second delivery phase.

In yet other examples, a system for using a hydraulic cartridge for eye surgery may comprise a nozzle, an implant bay coupled to the nozzle, an implant disposed in the implant bay, a housing coupled to the implant bay, a plunger configured to slide within the housing, a bore through the plunger, and a plunger rod having a threaded end disposed within the housing. A lead nut may be threaded onto the threaded end of the plunger rod, and a chamber may be configured to receive the hydraulic cartridge between the plunger and the lead nut. The plunger rod may be configured to be pushed or to otherwise slide to advance the lead nut, the hydraulic cartridge, and the plunger to advance the implant from the implant bay to the nozzle in a first delivery phase. The plunger rod may be configured to be rotated through the lead nut to drive a working fluid from the hydraulic cartridge through the bore to advance the implant through the nozzle in a second delivery phase.

Features, elements, and aspects described in the context of some embodiments may also be omitted, combined, or replaced by alternative features. Other features, objectives, advantages, and a preferred mode of making and using the claimed subject matter are described in greater detail below with reference to the accompanying drawings of illustrative embodiments.

### BRIEF DESCRIPTION OF THE DRAWINGS

The accompanying drawings illustrate some objectives, advantages, and a preferred mode of making and using some embodiments of the claimed subject matter. Like reference numbers represent like parts in the examples.
Figure 1 is a schematic diagram of an example system for delivering an implant into an eye.
Figure 2A and Figure 2B are schematic diagrams illustrating an example operation of the system of Figure 1.
Figure 3 is a schematic diagram of another example system for delivering an implant into an eye.
Figure 4A and Figure 4B are schematic diagrams illustrating an example operation of the system of Figure 3.
Figure 5A and Figure 5B are schematic diagrams further illustrating an example use of the system of Figure 1 or Figure 3 to deliver an implant to an eye.

### DESCRIPTION OF EXAMPLE EMBODIMENTS

The following description of example embodiments provides information that enables a person skilled in the art to make and use the subject matter set forth in the appended claims, but it may omit certain details already well known in the art. The following detailed description is, therefore, to be taken as illustrative and not limiting.

The example embodiments may also be described herein with reference to spatial relationships between various elements or to the spatial orientation of various elements depicted in the attached drawings. In general, such relationships or orientation assume a frame of reference consistent with or relative to a patient in a position to receive an implant. However, as should be recognized by those skilled in the art, this frame of reference is merely a descriptive expedient rather than a strict prescription.

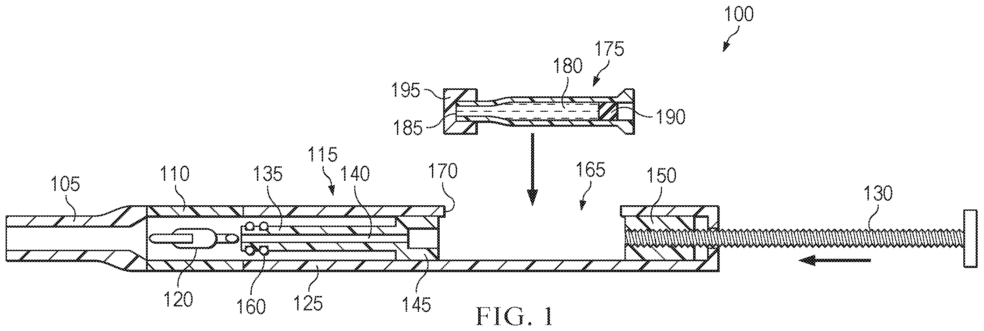

Figure 1 is a schematic diagram of a system 100 that can be used to deliver an implant into an eye. For example, as illustrated in Figure, 1, some embodiments of the system 100 may include a nozzle 105, an implant bay 110 that can be coupled to the nozzle 105, and an actuator 115 that can be coupled to the implant bay 110. Initially, various components of the system 100 may be assembled if needed. In the example of Figure 1, the nozzle 105, the implant bay 110, and the actuator 115 are fixed together to form a unitary structure. In other embodiments, the system 100 may comprise two or more modules, which can be configured to be coupled and decoupled as appropriate for storage, assembly, use, and disposal.

In general, components of the system 100 may be coupled directly or indirectly. For example, the nozzle 105 may be directly coupled to the implant bay 110 and may be indirectly coupled to the actuator 115 through the implant bay 110. Coupling may include fluid, mechanical, thermal, electrical, or chemical coupling (such as a chemical bond), or some combination of coupling in some contexts. For example, the actuator 115 may be mechanically and fluidly coupled to the nozzle 105. In some embodiments, components may also be coupled by virtue of physical proximity, being integral to a single structure, or being formed from the same piece of material.

The nozzle 105 generally comprises a tip adapted for insertion through an incision into an eye. The size of the tip may be adapted to surgical requirements and techniques as needed. For example, small incisions are generally preferable to reduce or minimize healing times. Incisions of less than 3 millimeters may be preferable in some instances, and the tip of the nozzle 105 may have a width of less than 3 millimeters in some embodiments.

The implant bay 110 generally represents a wide variety of apparatuses that are suitable for storing an implant prior to delivery into an eye. In Figure 1, for example, an implant 120 is disposed within the implant bay 110. In some embodiments, the implant 120 may comprise an intraocular lens having a shape similar to that of a natural lens of an eye, and it may be made from numerous materials. Examples of suitable materials may include silicone, acrylic, and combinations of such suitable materials. In some instances, the implant 120 may comprise an intraocular lens that is fluid-filled, such as a fluid-filled accommodating intraocular lens. The implant 120 may also comprise an intraocular lens that includes one or more features for positioning the intraocular lens within an eye. For example, the implant 120 may comprise one or more haptics, which can be oriented by the implant bay 110 prior to delivery.

In some embodiments, the implant bay 110 may additionally or alternatively be configured to prepare the implant 120 for delivery. For example, some embodiments of the implant bay 110 may be configured to be actuated by a surgeon or other operator to prepare the implant 120 for delivery by subsequent action of the actuator 115. In some instances, the implant bay 110 may be configured to actively deform, elongate, extend, or otherwise manipulate features of the implant 120 before the implant 120 is advanced into the nozzle 105. For example, some embodiments of the implant bay 110 may be configured to orient, fold, or splay an implant.

The actuator 115 of Figure 1 generally comprises a housing 125, a plunger rod 130, a plunger 135, and a bore 140 through the plunger 135. The plunger rod 130 and the plunger 135 are generally comprised of a substantially rigid material, such as a medical grade polymer material. The plunger 135 may additionally comprise a coupling 145. A lead nut 150 may be coupled to the plunger rod 130. A portion of the plunger 135 may extend into the implant bay 110.

In some embodiments, the actuator 115 may additionally comprise a nozzle seal 160. As illustrated in the example of Figure 1, the nozzle seal 160 may be a ring seal, such as an O-ring, disposed circumferentially around a portion of the plunger 135. In other examples, an umbrella seal may be suitable. In more particular embodiments, the nozzle seal 160 may be disposed distal to the coupling 145.

As illustrated in the example of Figure 1, the system 100 may also comprise a chamber 165. The chamber 165 of Figure 1 is generally formed between the plunger 135 and an end of the housing 125 and may be accessible through a slot 170 in the housing 125 or other suitable opening in the actuator 115. In some configurations, the plunger rod 130 may be advanced toward the plunger 135 to reduce the length of the system 100, which may be advantageous for transporting and storing the system 100. Before use, the plunger rod 130 may be retracted to open the chamber 165, as illustrated in the example of Figure 1.

The chamber 165 may be configured to receive a cartridge 175, which may contain a working fluid 180. Suitable working fluids may include, without limitation, a liquid, such as saline, or a viscous lubricant with non-Newtonian properties. The cartridge 175 may then be inserted into the chamber 165 to prepare the system 100 to deliver the implant 120. In other examples, the cartridge 175 may be transported and stored in the chamber 165. The cartridge 175 generally comprises a cartridge tip 185 and a cartridge seal 190. A cap 195 may be initially disposed over the cartridge tip 185. The cartridge seal 190 and the cap 195 together may contain the working fluid 180 within the cartridge 175, which may be suitable for transporting and storing the cartridge 175. In some embodiments, the cartridge 175 may comprise or consist essentially of a vial of working fluid. Suitable working fluids may include, without limitation, a liquid, such as saline, or a viscous lubricant with non-Newtonian properties.

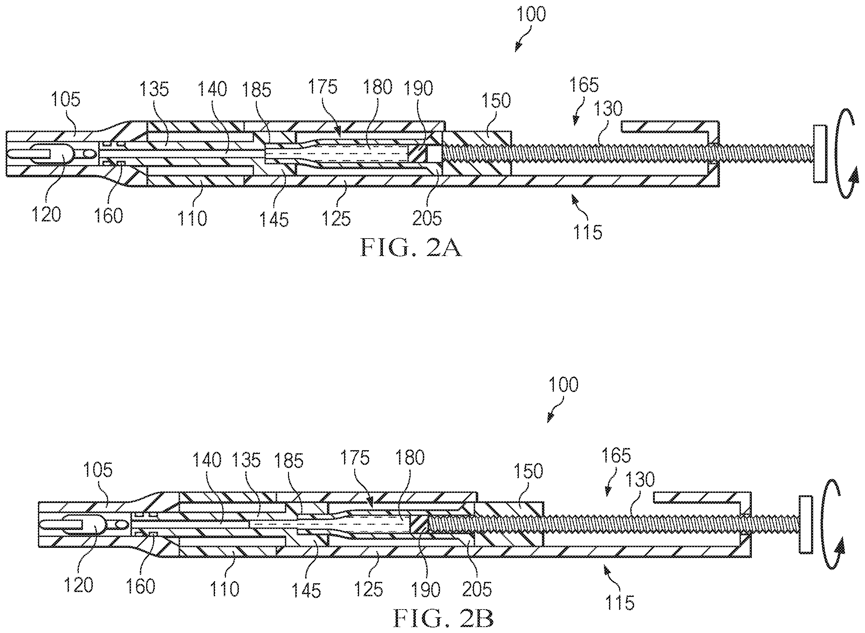

Figure 2A and Figure 2B are also schematic diagrams of the system 100, illustrating additional details that may be associated with the use of some embodiments. The actuator 115 is generally configured to allow the plunger 135 to advance the implant 120 mechanically from the implant bay 110 into the nozzle 105 in a first phase of delivery, as illustrated in the example of Figure 2A, and to allow the working fluid 180 to be moved from the cartridge 175 through the implant bay 110 and the nozzle 105 to advance the implant 120 fluidly or hydraulically through the nozzle 105 in a second phase, as illustrated in the example of Figure 2B.

For example, if assembled as illustrated in Figure 2A and Figure 2B, the implant bay 110 may be disposed between the nozzle 105 and the actuator 115. In the example of Figure 2A and Figure 2B, the cap 195 (not shown) has been removed from the cartridge tip 185, and the cartridge 175 has been inserted into the chamber 165. The actuator 115 may fluidly couple the working fluid 180 in the cartridge 175 to the implant bay 110 and/or the nozzle 105. For example, the coupling 145 may be configured to receive the cartridge tip 185 and to fluidly couple the working fluid 180 in the cartridge 175 to the implant bay 110 and the nozzle 105 through the bore 140, as illustrated in Figure 2A and Figure 2B. The plunger rod 130 may also be coupled to the cartridge 175. For example, the plunger rod 130, or a portion thereof, may be configured to slide within the housing 125 to push the cartridge 175, which in turn can push the plunger 135 and the implant 120 in the first delivery phase. In more particular examples, the lead nut 150 may engage the cartridge 175 opposite the cartridge tip 185 to advance the cartridge 175 through the chamber 165 as the plunger rod 130 is advanced.

In the example of Figure 2A, the cartridge 175 has been inserted into the chamber 165, and the plunger rod 130 has been pushed to advance the cartridge 175 and the plunger 135. As illustrated in Figure 2A, the lead nut 150 can maintain a relative position of the plunger rod 130 and the cartridge 175 as both advance through the housing 125 in the first delivery phase. For example, some embodiments of the plunger rod 130 may have a threaded end disposed within the housing 125, and the lead nut 150 may be threaded onto the threaded end of the plunger rod 130. If the plunger rod 130 is pushed, the lead nut 150 may apply force to a flange 205 of the cartridge 175 to rigidly move the cartridge 175 and the plunger 135 while maintaining the relative position of the plunger rod 130 and the cartridge seal 190, thereby allowing the working fluid 180 to be retained in the cartridge 175.

In the example of Figure 2A, the implant 120 has also been advanced into the nozzle 105 by plunger 135. The nozzle seal 160 has also been advanced into the nozzle 105 to create a seal in the nozzle 105 behind the implant 120. In some examples, the nozzle seal 160 may also prevent further advancement of the plunger 135 and the cartridge 175. In other examples, the system 100 may have a plunger stop configured to prevent further advancement. The implant 120 may also form a seal within the nozzle 105 in some instances. In the configuration of Figure 2A, the bore 140 may fluidly couple the working fluid 180 in the cartridge 175 to the nozzle 105.

As illustrated in the example of Figure 2B, with the implant 120 advanced into the nozzle 105, the plunger rod 130 may be advanced from the position of Figure 2A to move the working fluid 180 from the cartridge 175 through the plunger 135 in a second phase of delivery. For example, the plunger rod 130 may be twisted to advance a distal end of the plunger rod 130 through threads in the lead nut 150, while the housing 125 can prevent rotation of the lead nut 150. Advancing the plunger rod 130 can advance the cartridge seal 190, which can force the working fluid 180 through the bore 140 into the nozzle 105 behind the implant 120. Movement of the working fluid 180 from the bore 140 into the nozzle 105 under pressure from the cartridge seal 190 can increase the pressure and flow rate of the working fluid 180 in the nozzle 105 behind the implant 120, which can advance the implant 120 further through the nozzle 105 until the implant 120 is ejected from the nozzle 105.

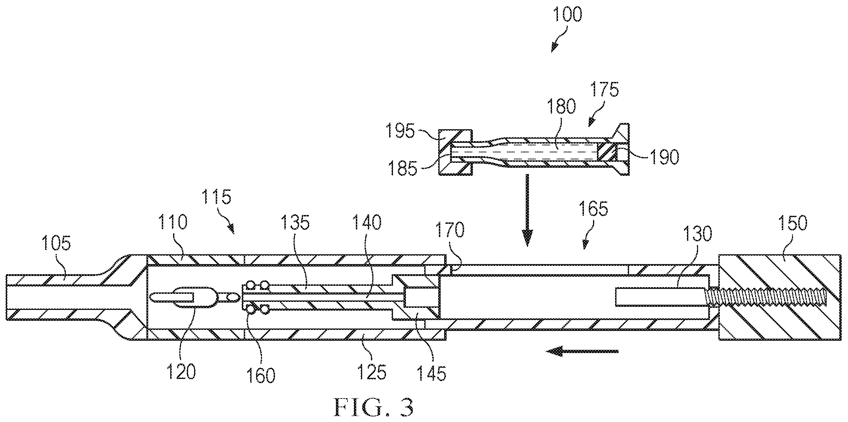

Figure 3 is a schematic diagram of another example of the system 100, which can be used to deliver an implant into an eye. The example of Figure 3 is similar or analogous to the example of Figure 1 in several respects. For example, the system 100 of Figure 3 may include the nozzle 105, the implant bay 110, and the actuator 115.

The actuator 115 of Figure 3 generally comprises the housing 125, the plunger rod 130, the plunger 135, and the bore 140 through the plunger 135. The plunger 135 may additionally comprise the coupling 145. In the example of Figure 3, the lead nut 150 may be coupled to a threaded portion of the plunger rod 130 and coupled to an end of the housing 125. In more particular examples, a threaded portion of the plunger rod may extend through a wall in the chamber 165, and the lead nut 150 may be coupled to the chamber 165. A portion of the plunger 135 may extend into the implant bay 110.

The chamber 165 of Figure 3 is generally formed between the plunger 135 and the lead nut 150 and may be accessible through the slot 170 or other suitable opening in the actuator 115. In some configurations, the chamber 165 may be advanced toward the plunger 135 to reduce the length of the system 100, which may be advantageous for transporting and storing the system 100. In Figure 3, for example, a portion of the chamber 165 may slide over at least a portion of the plunger 135, between the housing 125 and the plunger 135. Before use, the chamber 165 may be retracted to expose the slot 170, as illustrated in the example of Figure 3.

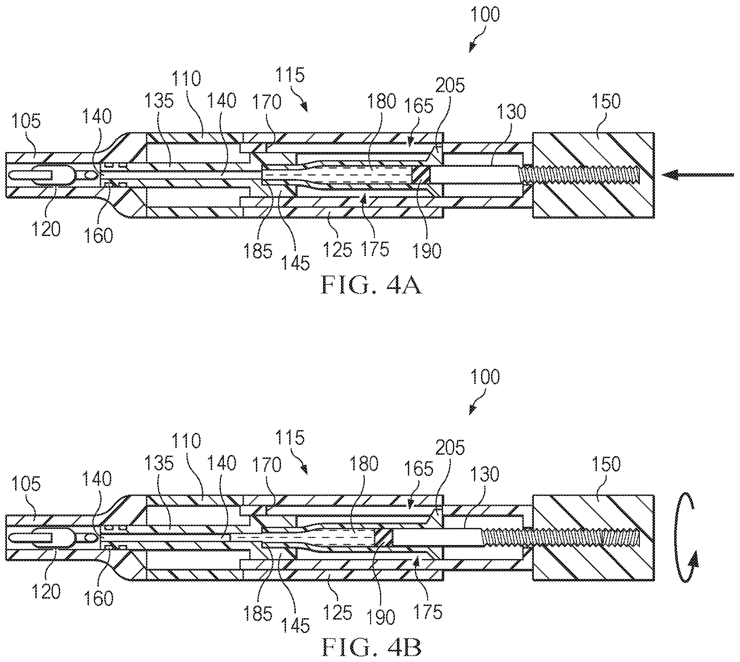

Figure 4A and Figure 4B are also schematic diagrams of the system 100 of Figure 3, illustrating additional details that may be associated with the use of some embodiments. The actuator 115 is generally configured to allow the plunger 135 to advance the implant 120 mechanically from the implant bay 110 into the nozzle 105 in a first phase of delivery, as illustrated in the example of Figure 4A, and to allow the working fluid 180 to be moved from the cartridge 175 through the implant bay 110 and the nozzle 105 to advance the implant 120 fluidly or hydraulically through the nozzle 105 in a second phase, as illustrated in the example of Figure 4B.

For example, if assembled as illustrated in Figure 4A and Figure 4B, the implant bay 110 may be disposed between the nozzle 105 and the actuator 115. In the example of Figure 4A and Figure 4B, the cap 195 (not shown) has been removed from the cartridge tip 185, and the cartridge 175 has been inserted into the chamber 165. The actuator 115 may fluidly couple the working fluid 180 in the cartridge 175 to the implant bay 110 and/or the nozzle 105. For example, the coupling 145 may be configured to receive the cartridge tip 185 and to fluidly couple the working fluid 180 in the cartridge 175 to the implant bay 110 and the nozzle 105 through the bore 140, as illustrated in Figure 4A and Figure 4B. The plunger rod 135 may be coupled to cartridge seal 190. The chamber 165, or a portion thereof, may be configured to slide within the housing 125 to push the cartridge 175, which in turn can push the plunger 135 and the implant 120 in the first delivery phase. In more particular examples, the slot 170 may engage the cartridge 175 opposite the cartridge tip 185.

In the example of Figure 4A, the cartridge 175 has been inserted into the chamber 165, and the chamber 165 has been pushed to advance the cartridge 175 and the plunger 135. As illustrated in Figure 4A, the lead nut 150 can maintain a relative position of the plunger rod 130 and the cartridge 175 as both advance through the housing 125 in the first delivery phase. For example, some embodiments of the plunger rod 130 may have a threaded end disposed within the lead nut 150, and the lead nut 150 may be coupled to an end of the chamber 165. If the lead nut 150 is pushed, the slot 170 may apply force to the flange 205 of the cartridge 175 to rigidly move the cartridge 175 and the plunger 135 while maintaining the relative position of the plunger rod 130 and the cartridge seal 190, thereby allowing the working fluid 180 to be retained in the cartridge 175.

In the example of Figure 4A, the implant 120 has also been advanced into the nozzle 105 by plunger 135. The nozzle seal 160 may also be advanced into the nozzle 105 to create a seal in the nozzle 105 behind the implant 120. In some examples, the nozzle seal 160 may also prevent further advancement of the plunger 135 and the cartridge 175. In other examples, the system 100 may have a plunger stop configured to prevent further advancement. The implant 120 may also form a seal within the nozzle 105 in some instances. In the configuration of Figure 4A, the bore 140 may fluidly couple the working fluid 180 in the cartridge 175 to the nozzle 105.

As illustrated in the example of Figure 4B, with the implant 120 advanced into the nozzle 105, the plunger rod 130 may be advanced from the position of Figure 4A to move the working fluid 180 from the cartridge 175 through the plunger 135 in a second phase of delivery. For example, the lead nut 150 may be twisted to advance the plunger rod 130 through threads in the lead nut 150, while the chamber 165 can prevent rotation of the plunger rod 130. Advancing the plunger rod 130 can advance the cartridge seal 190, which can force the working fluid 180 through the bore 140 into the nozzle 105 behind the implant 120. Movement of the working fluid 180 from the bore 140 into the nozzle 105 under pressure from the cartridge seal 190 can increase the pressure and flow rate of the working fluid 180 in the nozzle 105 behind the implant 120, which can advance the implant 120 further through the nozzle 105 until the implant 120 is ejected from the nozzle 105.

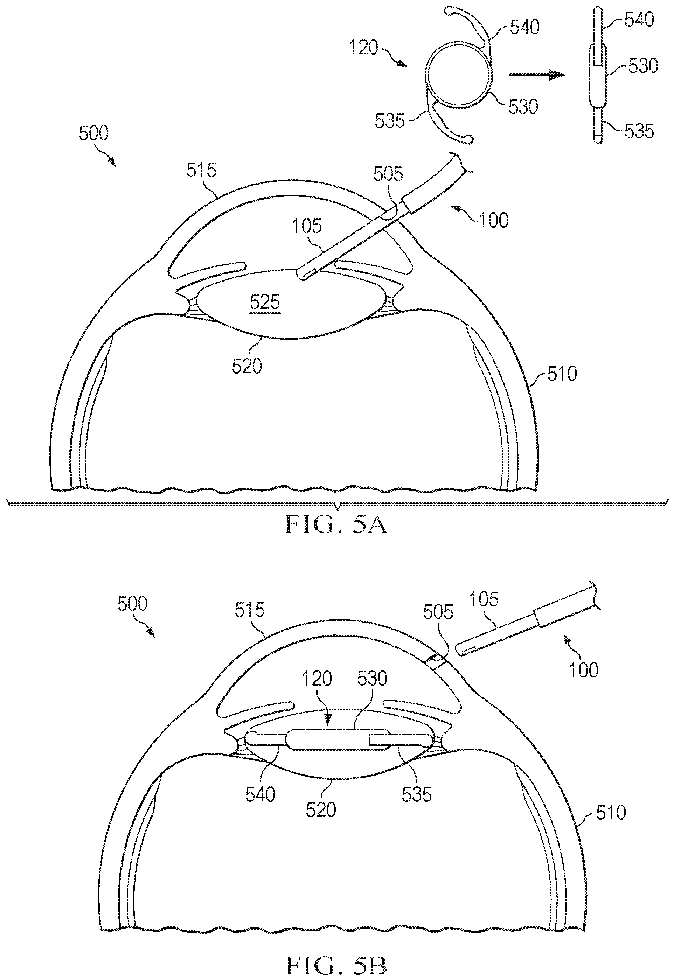

Figures 5A-5B are schematic diagrams further illustrating an example use of the system 100 to deliver the implant 120 to an eye 500. As illustrated, an incision 505 may be made in the eye 500 by a surgeon, for example. In some instances, the incision 505 may be made through the sclera 510 of the eye 500. In other instances, an incision may be formed in the cornea 515 of the eye 500. The incision 505 may be sized to permit insertion of a portion of the nozzle 105 to deliver the implant 120 into the capsular bag 520. For example, in some instances, the size of the incision 505 may have a length less than about 3000 microns (3 millimeters). In other instances, the incision 505 may have a length of from about 1000 microns to about 1500 microns, from about 1500 microns to about 2000 microns, from about 2000 microns to about 2500 microns, or from about 2500 microns to about 3000 microns.

After the incision 505 is made, the nozzle 105 can be inserted through the incision 505 so that the tip of the nozzle 105 aligns with the incision 505, allowing the nozzle 105 to extend into an interior portion 525 of the eye 500. The system 100 can then eject the implant 120 through the nozzle 105 into the capsular bag 520 of the eye 500, substantially as described above with reference to Figures 2A-2B or Figures 4A-4B.

In some embodiments, the implant 120 may comprise an intraocular lens. In some instances, the implant 120 may comprise an intraocular lens that is fluid-filled, such as a fluid-filled accommodating intraocular lens. The implant 120 may also comprise an intraocular lens that includes one or more features, such as haptics, for positioning the intraocular lens within an eye. In the example of Figure 5A and Figure 5B, the implant 120 is illustrative of an intraocular lens having an optic body 530, a leading haptic 535, and a trailing haptic 540.

In some applications, the implant 120 may be delivered in a folded, straightened, or splayed configuration and can revert to an initial, resting state, within the capsular bag 520, as shown in Figure 5B. The capsular bag 520 can retain the implant 120 within the eye 500 in a relationship relative to the eye 500 so that the optic body 530 refracts light directed to the retina (not shown). The leading haptic 535 and the trailing haptic 540 can engage the capsular bag 520 to secure the implant 120 therein. After dispensing the implant 120 into the capsular bag 520, the nozzle 105 may be removed from the eye 500 through the incision 505, and the eye 500 can be allowed to heal over time.

The systems, apparatuses, and methods described herein may provide significant advantages. For example, some embodiments may be particularly advantageous for delivering intraocular lenses, including fluid-filled accommodating lenses, which can present unique challenges for delivery. Some embodiments can compress a relatively large lens to fit through an acceptably small incision, manage deformation caused by shifting fluid during compression and exit from a nozzle, and execute delivery in a predictable and controlled manner. Additionally, some embodiments can reduce system complexity and the number of delivery steps while maintaining haptic position consistency. Some embodiments may also reduce the amount of working fluid for delivery. For example, a single vial of ophthalmic viscosurgical device (OVD), such as vial of CELLUGEL OVD, PROVISC OVD, OR DISCOVISC OVD, may be used to drive some embodiments of the system 100 and provide the working fluid for delivery. Additionally, or alternatively, some embodiments may be particularly advantageous for providing multiple delivery modes in a single apparatus. For example, a first delivery mode can allow an operator to advance an implant to a dwell position by pushing a plunger rod. A second delivery mode can allow an operator to twist the plunger rod to advance an implant, which can give the operator finer position control as the implant is delivered.

While shown in a few illustrative embodiments, a person having ordinary skill in the art will recognize that the systems, apparatuses, and methods described herein are susceptible to various changes and modifications that fall within the scope of the appended claims. Moreover, descriptions of various alternatives using terms such as "or" do not require mutual exclusivity unless clearly required by the context, and the indefinite articles "a" or "an" do not limit the subject to a single instance unless clearly required by the context. Components may also be combined or eliminated in various configurations for purposes of sale, manufacture, assembly, or use. For example, in some configurations, the nozzle 105, the implant bay 110, and the actuator 115 may each be separated from one another or combined in various ways for manufacture or sale.

## Claims

1. A system (100) for delivering an implant using a cartridge for eye surgery, the system comprising:
a nozzle (105);
an implant bay (110) coupled to the nozzle; and
an actuator (115) comprising:
a plunger (135) and a chamber (165) configured to receive the cartridge (175);
a plunger rod (130), and
a bore (140) through the plunger (135);
wherein the actuator (115) is configured to move the plunger (135) to advance an implant (120) from the implant bay (110) into the nozzle (105), to fluidly couple a working fluid (180) in the cartridge (175) to the implant bay (110), and to move the working fluid (180) from the cartridge (175) through the plunger (135) to advance the implant through the nozzle; and
wherein the plunger rod (130) is configured to move the working fluid (180) from the cartridge (175) through the bore (140) to advance the implant (120) through the nozzle (105).

2. The system of claim 1, wherein the plunger (135) comprises a coupling (145) configured to fluidly couple the cartridge (175) to the implant bay (110).

3. The system of claim 1, further comprising a lead nut (150) wherein the lead nut is arranged according to one or more of the following:
(i) wherein the lead nut (150) is threaded onto the plunger rod (130), wherein the lead nut (150) is configured to advance the plunger rod (130) to move the working fluid;
(ii) wherein the lead nut (150) is configured to maintain a relative position of the plunger rod (130) and the cartridge (175) as the implant (120) is advanced from the implant bay (110) to the nozzle (105).

4. The system of claim 3, wherein:
the cartridge (175) comprises a cartridge seal (190); and
the plunger rod (130) is configured to advance the cartridge seal (190) to move the working fluid (180).

5. The system of claim 4, wherein:
the lead nut (150) is configured to advance the cartridge (175) through the chamber (165); and the plunger rod (130) is configured to be rotated through the lead nut (150) to advance the cartridge seal.

6. The system of claim 4, wherein:
the chamber is configured to advance the cartridge (175) and the plunger (135);
the lead nut (150) is coupled to an end of the chamber (165); and
the lead nut (150) is configured to be rotated around the plunger rod (130) to advance the cartridge seal (190).

7. The system of claim 1, wherein the cartridge is a hydraulic cartridge, further comprising:
an implant (120) disposed in the implant bay (110);
a housing (125) coupled to the implant bay (110);
wherein:
the plunger (135) is disposed within the housing (125);
the bore (140) through the plunger (135) is fluidly coupled to the implant bay (110);
the plunger rod (130) is at least partially disposed within the housing (125); and
the chamber (165) configured to receive the hydraulic cartridge (175) between the plunger (135) and the plunger rod (130); and
wherein the plunger (135) is configured to advance the implant (120) from the implant bay (110) to the nozzle (105) in a first delivery phase, and the plunger rod (125) is operable to drive a working fluid (180) from the hydraulic cartridge (175) through the bore (140) to advance the implant (120) through the nozzle (105) in a second delivery phase.

8. The system of claim 7, further comprising a lead nut, wherein the lead nut (150) is arranged according to one or more of the following:
(i) wherein the lead nut (150) is threaded onto the plunger rod (130), wherein the lead nut (150) is configured to advance the plunger rod (135) to drive the working fluid (180) through the bore (140);
(ii) wherein the lead nut (150) is configured to maintain a relative position of the plunger rod (130) and the hydraulic cartridge (175) as the implant (120) is advanced from the implant bay (110) to the nozzle (105).

9. The system of claim 8, wherein:
the hydraulic cartridge (175) comprises a cartridge seal (190); and
the plunger rod (130) is configured to be rotated through the lead nut (150) to advance the cartridge seal (190) to drive the working fluid (180).

10. The system of claim 9, wherein:
the chamber (165) is configured to advance the cartridge (175) and the plunger (135);
the lead nut (150) is coupled to an end of the chamber (165); and
the lead nut (150) is configured to be rotated around the plunger rod (30) to advance the cartridge seal (190).

11. The system of claim 1, wherein the cartridge is a hydraulic cartridge, further comprising:
an implant (120) disposed in the implant bay (110);
a housing (125) coupled to the implant bay (110);
wherein:
the plunger (135) is configured to slide within the housing (125); the bore (140) through the plunger (135) fluidly coupled to the implant bay (110);
the plunger rod (130) having a threaded end disposed within the housing (125); and
further comprising:
a lead nut (150) threaded onto the threaded end of the plunger rod (130); and
wherein the chamber (165) is configured to receive the hydraulic cartridge (175) between the plunger (135) and the lead nut (150);
wherein the plunger rod (130) is configured to be pushed to rigidly move the lead nut (150), the hydraulic cartridge (175), and the plunger (130) to advance the implant (120) from the implant bay (110) to the nozzle (105), and the plunger rod (130) is configured to be rotated through the lead nut (150) to drive a working fluid (180) from the hydraulic cartridge (175) through the bore (140) to advance the implant (120) through the nozzle (105).

12. The system of claim 7 or the system of claim 11, wherein the plunger (135) comprises a coupling (145) configured to receive the hydraulic cartridge (175) and to fluidly couple the hydraulic cartridge (175) to the bore (140).

13. The system of claim 11 or claim 12, wherein the lead nut (150) is configured to maintain a relative position of the plunger rod (130) and the hydraulic cartridge (175) as the implant (120) is advanced from the implant bay (110) to the nozzle (105).

14. The system of any of claims 11-13, wherein:
the hydraulic cartridge (175) comprises a cartridge seal (190); and
the plunger rod (130) is configured to advance the cartridge seal (190) to drive the working fluid from the hydraulic cartridge (175) through the bore (140).

15. The system according to claim 1, further comprising:
an implant (120) disposed in the implant bay (110);
a housing (125) coupled to the implant bay (110);
wherein:
the plunger (135) is configured to slide within the housing (125);
the bore (140) through the plunger (135) is fluidly coupled to the implant bay (110);
the plunger rod (130) having a threaded end disposed within the housing (125);
further comprising:
a lead nut (150) threaded onto the threaded end of the plunger rod (130);
wherein the chamber (165) is between the plunger (135) and the lead nut (150); and
wherein the cartridge (175) is disposed in the chamber (165), the cartridge (175) comprising a tip (185) fluidly coupled to the bore (140) through the plunger (135), a cartridge seal (190), and a working fluid disposed between the tip (185) and the cartridge seal (190);
wherein the plunger rod (130) is configured to be pushed to advance the lead nut (150), the cartridge (175), the plunger (135), and the implant (120) from the implant bay (110) to the nozzle (105), and the plunger rod (135) is configured to be rotated through the lead nut (150) to advance the cartridge seal (190), thereby driving the working fluid (180) through the bore (140) to advance the implant (120) through the nozzle (105).

16. A system for delivering an implant using a hydraulic cartridge for eye surgery, the system comprising:
a nozzle (105);
an implant bay (110) coupled to the nozzle (105);
an implant (120) disposed in the implant bay (110);
a housing (125) coupled to the implant bay (110);
a plunger (135) disposed within the housing (125);
a bore (140) through the plunger (135), the bore (140) fluidly coupled to the implant bay (110);
a plunger rod (135) at least partially disposed within the housing (125); and
a chamber (165) configured to receive the hydraulic cartridge (175) between the plunger (135) and the plunger rod (130);
wherein the chamber (165) is configured to advance the implant (120) from the implant bay (110) to the nozzle (105) in a first delivery phase, and the plunger rod (130) is operable to drive a working fluid (180) from the hydraulic cartridge (175) through the bore (140) to advance the implant (120) through the nozzle (105) in a second delivery phase.

## Patentansprüche

1. System (100) zum Abgeben eines Implantats unter Verwendung einer Kartusche für Augenchirurgie, wobei das System umfasst:
eine Düse (105);
einen Implantatschacht (110), der mit der Düse gekoppelt ist; und
einen Aktuator (115), umfassend:
einen Kolben (135) und eine Kammer (165), die dazu ausgelegt ist, die Kartusche (175) aufzunehmen;
eine Kolbenstange (130), und
eine Bohrung (140) durch den Kolben (135);
wobei der Aktuator (115) dazu ausgelegt ist, den Kolben (135) zu bewegen, um ein Implantat (120) aus dem Implantatschacht (110) in die Düse (105) vorzuschieben, ein Arbeitsfluid (180) in der Kartusche (175) fluidmäßig mit dem Implantatschacht (110) zu koppeln und das Arbeitsfluid (180) aus der Kartusche (175) durch den Kolben (135) zu bewegen, um das Implantat durch die Düse hindurch vorzuschieben; und
wobei die Kolbenstange (130) dazu ausgelegt ist, das Arbeitsfluid (180) aus der Kartusche (175) durch die Bohrung (140) zu bewegen, um das Implantat (120) durch die Düse (105) hindurch vorzuschieben.

2. System nach Anspruch 1, wobei der Kolben (135) eine Kupplung (145) umfasst, die dazu ausgelegt ist, die Kartusche (175) fluidmäßig mit dem Implantatschacht (110) zu koppeln.

3. System nach Anspruch 1, ferner umfassend eine Spindelmutter (150), wobei die Spindelmutter gemäß einem oder mehreren der Folgenden angeordnet ist:
(i) wobei die Spindelmutter (150) auf die Kolbenstange (130) geschraubt ist, wobei die Spindelmutter (150) dazu ausgelegt ist, die Kolbenstange (130) vorzuschieben, um das Arbeitsfluid zu bewegen;
(ii) wobei die Spindelmutter (150) dazu ausgelegt ist, eine relative Position der Kolbenstange (130) und der Kartusche (175) beizubehalten, wenn das Implantat (120) aus dem Implantatschacht (110) zu der Düse (105) vorgeschoben wird.

4. System nach Anspruch 3, wobei:
die Kartusche (175) eine Kartuschendichtung (190) umfasst; und
die Kolbenstange (130) dazu ausgelegt ist, die Kartuschendichtung (190) vorzuschieben, um das Arbeitsfluid (180) zu bewegen.

5. System nach Anspruch 4, wobei:
die Spindelmutter (150) dazu ausgelegt ist, die Kartusche (175) durch die Kammer (165) vorzuschieben; und die Kolbenstange (130) dazu ausgelegt ist, durch die Spindelmutter (150) gedreht zu werden, um die Kartuschendichtung vorzuschieben.

6. System nach Anspruch 4, wobei:
die Kammer dazu ausgelegt ist, die Kartusche (175) und den Kolben (135) vorzuschieben;
die Spindelmutter (150) an ein Ende der Kammer (165) gekoppelt ist; und
die Spindelmutter (150) dazu ausgelegt ist, um die Kolbenstange (130) gedreht zu werden, um die Kartuschendichtung (190) vorzuschieben.

7. System nach Anspruch 1, wobei die Kartusche eine hydraulische Kartusche ist, ferner umfassend:
ein Implantat (120), das in dem Implantatschacht (110) angeordnet ist;
ein Gehäuse (125), das mit dem Implantatschacht (110) gekoppelt ist;
wobei:
der Kolben (135) innerhalb des Gehäuses (125) angeordnet ist;
die Bohrung (140) durch den Kolben (135) fluidmäßig an den Implantatschacht (110) gekoppelt ist;
die Kolbenstange (130) mindestens teilweise innerhalb des Gehäuses (125) angeordnet ist; und
die Kammer (165) dazu ausgelegt ist, die hydraulische Kartusche (175) zwischen dem Kolben (135) und der Kolbenstange (130) aufzunehmen; und
wobei der Kolben (135) dazu ausgelegt ist, das Implantat (120) in einer ersten Abgabephase aus dem Implantatschacht (110) zu der Düse (105) vorzuschieben, und in einer zweiten Abgabephase die Kolbenstange (125) betreibbar ist, um ein Arbeitsfluid (180) aus der hydraulischen Kartusche (175) durch die Bohrung (140) zu treiben, um das Implantat (120) durch die Düse (105) hindurch vorzuschieben.

8. System nach Anspruch 7, ferner umfassend eine Spindelmutter, wobei die Spindelmutter (150) gemäß einem oder mehreren der Folgenden angeordnet ist:
(i) wobei die Spindelmutter (150) auf die Kolbenstange (130) geschraubt ist, wobei die Spindelmutter (150) dazu ausgelegt ist, die Kolbenstange (135) vorzuschieben, um das Arbeitsfluid (180) durch die Bohrung (140) zu treiben;
(ii) wobei die Spindelmutter (150) dazu ausgelegt ist, eine relative Position der Kolbenstange (130) und der hydraulischen Kartusche (175) beizubehalten, wenn das Implantat (120) aus dem Implantatschacht (110) zu der Düse (105) vorgeschoben wird.

9. System nach Anspruch 8, wobei:
die hydraulische Kartusche (175) eine Kartuschendichtung (190) umfasst; und
die Kolbenstange (130) dazu ausgelegt ist, durch die Spindelmutter (150) gedreht zu werden, um die Kartuschendichtung (190) vorzuschieben, um das Arbeitsfluid (180) zu treiben.

10. System nach Anspruch 9, wobei:
die Kammer (165) dazu ausgelegt ist, die Kartusche (175) und den Kolben (135) vorzuschieben;
die Spindelmutter (150) an ein Ende der Kammer (165) gekoppelt ist; und
die Spindelmutter (150) dazu ausgelegt ist, um die Kolbenstange (30) gedreht zu werden, um die Kartuschendichtung (190) vorzuschieben.

11. System nach Anspruch 1, wobei die Kartusche eine hydraulische Kartusche ist, ferner umfassend:
ein Implantat (120), das in dem Implantatschacht (110) angeordnet ist;
ein Gehäuse (125), das mit dem Implantatschacht (110) gekoppelt ist;
wobei:
der Kolben (135) dazu ausgelegt ist, innerhalb des Gehäuses (125) zu gleiten; die Bohrung (140) durch den Kolben (135) fluidmäßig mit dem Implantatschacht (110) gekoppelt ist;
die Kolbenstange (130) ein Gewindeende aufweist, das innerhalb des Gehäuses (125) angeordnet ist; und
ferner umfassend:
eine Spindelmutter (150), die auf das Gewindeende der Kolbenstange (130) geschraubt ist; und
wobei die Kammer (165) dazu ausgelegt ist, die hydraulische Kartusche (175) zwischen dem Kolben (135) und der Spindelmutter (150) aufzunehmen;
wobei die Kolbenstange (130) dazu ausgelegt ist, geschoben zu werden, um die Spindelmutter (150), die hydraulische Kartusche (175) und den Kolben (130) starr zu bewegen, um das Implantat (120) aus dem Implantatschacht (110) zu der Düse (105) vorzuschieben,
und die Kolbenstange (130) dazu ausgelegt ist, durch die Spindelmutter (150) gedreht zu werden, um ein Arbeitsfluid (180) aus der hydraulischen Kartusche (175) durch die Bohrung (140) zu treiben, um das Implantat (120) durch die Düse (105) hindurch vorzuschieben.

12. System nach Anspruch 7 oder System nach Anspruch 11, wobei der Kolben (135) eine Kupplung (145) umfasst, die dazu ausgelegt ist, die hydraulische Kartusche (175) aufzunehmen und die hydraulische Kartusche (175) fluidmäßig mit der Bohrung (140) zu koppeln.

13. System nach Anspruch 11 oder Anspruch 12, wobei die Spindelmutter (150) dazu ausgelegt ist, eine relative Position der Kolbenstange (130) und der hydraulischen Kartusche (175) beizubehalten, wenn das Implantat (120) aus dem Implantatschacht (110) zu der Düse (105) vorgeschoben wird.

14. System nach einem der Ansprüche 11-13, wobei:
die hydraulische Kartusche (175) eine Kartuschendichtung (190) umfasst; und
die Kolbenstange (130) dazu ausgelegt ist, die Kartuschendichtung (190) vorzuschieben, um das Arbeitsfluid aus der hydraulischen Kartusche (175) durch die Bohrung (140) zu treiben.

15. System nach Anspruch 1, ferner umfassend:
ein Implantat (120), das in dem Implantatschacht (110) angeordnet ist;
ein Gehäuse (125), das mit dem Implantatschacht (110) gekoppelt ist;
wobei:
der Kolben (135) dazu ausgelegt ist, innerhalb des Gehäuses (125) zu gleiten;
die Bohrung (140) durch den Kolben (135) fluidmäßig an den Implantatschacht (110) gekoppelt ist;
die Kolbenstange (130) ein Gewindeende aufweist, das innerhalb des Gehäuses (125) angeordnet ist;
ferner umfassend:
eine Spindelmutter (150), die auf das Gewindeende der Kolbenstange (130) geschraubt ist;
wobei sich die Kammer (165) zwischen dem Kolben (135) und der Spindelmutter (150) befindet; und
wobei die Kartusche (175) in der Kammer (165) angeordnet ist, wobei die Kartusche (175) eine Spitze (185), die fluidmäßig durch den Kolben (135) mit der Bohrung (140) gekoppelt ist, eine Kartuschendichtung (190) und ein Arbeitsfluid umfasst, das zwischen der Spitze (185) und
der Kartuschendichtung (190) angeordnet ist;
wobei die Kolbenstange (130) dazu ausgelegt ist, geschoben zu werden, um die Spindelmutter (150), die Kartusche (175), den Kolben (135) und das Implantat (120) aus dem Implantatschacht (110) zu der Düse (105) vorzuschieben, und die Kolbenstange (135) dazu ausgelegt ist, durch die Spindelmutter (150) gedreht zu werden, um die Kartuschendichtung (190) vorzuschieben, wodurch das Arbeitsfluid (180) durch die Bohrung (140) getrieben wird, um das Implantat (120) durch die Düse (105) hindurch vorzuschieben.

16. System zum Abgeben eines Implantats unter Verwendung einer hydraulischen Kartusche für die Augenchirurgie, wobei das System Folgendes umfasst:
eine Düse (105);
einen Implantatschacht (110), der mit der Düse (105) gekoppelt ist;
ein Implantat (120), das in dem Implantatschacht (110) angeordnet ist;
ein Gehäuse (125), das mit dem Implantatschacht (110) gekoppelt ist;
einen Kolben (135), der innerhalb des Gehäuses (125) angeordnet ist;
eine Bohrung (140) durch den Kolben (135), wobei die Bohrung (140) fluidmäßig an den Implantatschacht (110) gekoppelt ist;
eine Kolbenstange (135), die mindestens teilweise innerhalb des Gehäuses (125) angeordnet ist; und
eine Kammer (165), die dazu ausgelegt ist, die hydraulische Kartusche (175) zwischen dem Kolben (135) und der Kolbenstange (130) aufzunehmen;
wobei die Kammer (165) dazu ausgelegt ist, das Implantat (120) in einer ersten Abgabephase aus dem Implantatschacht (110) zu der Düse (105) vorzuschieben,
und in einer zweiten Abgabephase die Kolbenstange (130) betreibbar ist, um ein Arbeitsfluid (180) aus der hydraulischen Kartusche (175) durch die Bohrung (140) zu treiben, um das Implantat (120) durch die Düse (105) hindurch vorzuschieben.

## Revendications

1. Système d'administration (100) d'un implant à l'aide d'une cartouche pour chirurgie oculaire, le système comprenant :
une buse (105) ;
un compartiment à implant (110) couplé à la buse ; et
un actionneur (115) comportant :
un piston (135) et une chambre (165) configurée pour recevoir la cartouche (175) ;
une tige de piston (130), et
un alésage (140) à travers le piston (135) ;
l'actionneur (115) étant configuré pour déplacer le piston (135) afin de faire avancer un implant (120) depuis le compartiment d'implant (110) dans la buse (105), pour coupler fluidiquement un fluide de travail (180) dans la cartouche (175) au compartiment d'implant (110), et pour déplacer le fluide de travail (180) depuis la cartouche (175) à travers le piston (135) pour faire avancer l'implant à travers la buse ; et
la tige de piston (130) étant configurée pour déplacer le fluide de travail (180) depuis la cartouche (175) à travers l'alésage (140) afin de faire avancer l'implant (120) à travers la buse (105).

2. Système selon la revendication 1, le piston (135) comprenant un accouplement (145) configuré pour coupler fluidiquement la cartouche (175) au compartiment d'implant (110).

3. Système selon la revendication 1, comprenant en outre un écrou de guidage (150) l'écrou de guidage étant agencé selon un ou plusieurs des éléments suivants :
(i) l'écrou de guidage (150) étant enfilé sur la tige de piston (130), l'écrou de guidage (150) étant configuré pour faire avancer la tige de piston (130) afin de déplacer le fluide de travail ;
(ii) l'écrou de guidage (150) étant configuré pour maintenir une position relative de la tige de piston (130) et de la cartouche (175) lorsque l'implant (120) est avancé du compartiment d'implant (110) à la buse (105

4. Système selon la revendication 3,
la cartouche (175) comprenant un joint d'étanchéité de cartouche (190) ; et
la tige de piston (130) étant configurée pour faire avancer le joint de cartouche (190) afin de déplacer le fluide de travail (180).

5. Système selon la revendication 4, dans lequel :
l'écrou de guidage (150) étant configuré pour faire avancer la cartouche (175) à travers la chambre (165) ;
et la tige de piston (130) étant configurée pour tourner à travers l'écrou de guidage (150) afin de faire avancer le joint de cartouche.

6. Système selon la revendication 4, dans lequel :
la chambre étant configurée pour faire avancer la cartouche (175) et le piston (135) ;
l'écrou de guidage (150) étant couplé à une extrémité de la chambre (165) ; et
l'écrou de guidage (150) étant configuré pour tourner autour de la tige de piston (130) afin de faire avancer le joint de cartouche (190).

7. Système selon la revendication 1, la cartouche étant une cartouche hydraulique, comprenant en outre :
un implant (120) disposé dans le compartiment d'implants (110) ;
un boîtier (125) couplé au compartiment d'implants (110) ;
dans lequel :
le piston (135) est disposé à l'intérieur du boîtier (125) ;
l'alésage (140) à travers le piston (135) est couplé fluidiquement au compartiment d'implant (110) ;
la tige de piston (130) est au moins partiellement disposée à l'intérieur du boîtier (125) ; et
la chambre (165) configurée pour recevoir la cartouche hydraulique (175) entre le piston (135) et la tige de piston (130) ; et
le piston (135) étant configuré pour faire avancer l'implant (120) du compartiment d'implant (110) à la buse (105) dans une première phase d'administration, et
la tige de piston (125) étant actionnable pour entraîner un fluide de travail (180) de la cartouche hydraulique (175) à travers l'alésage (140) pour faire avancer l'implant (120) à travers la buse (105) dans une seconde phase d'administration.

8. Système selon la revendication 7, comprenant en outre un écrou de guidage (150), l'écrou de guidage étant agencé selon un ou plusieurs des cas suivants :
(i) l'écrou de guidage (150) étant enfilé sur la tige de piston (130), l'écrou de guidage (150) étant configuré pour faire avancer la tige de piston (135) pour entraîner le fluide de travail (180) à travers l'alésage (140) ;
(ii) l'écrou de guidage (150) étant configuré pour maintenir une position relative de la tige de piston (130) et de la cartouche hydraulique (175) lorsque l'implant (120) est avancé du compartiment d'implant (110) à la buse (105).

9. Système selon la revendication 8,
la cartouche hydraulique (175) comprenant un joint d'étanchéité de cartouche (190) ; et
la tige de piston (130) étant configurée pour tourner à travers l'écrou de guidage (150) afin de faire avancer le joint de cartouche (190) pour entraîner le fluide de travail (180).

10. Système selon la revendication 9,
la chambre (165) étant configurée pour faire avancer la cartouche (175) et le piston (135) ;
l'écrou de guidage (150) étant couplé à une extrémité de la chambre (165) ; et
l'écrou de guidage (150) étant configuré pour tourner autour de la tige de piston (30) afin de faire avancer le joint de cartouche (190).

11. Système selon la revendication 1, la cartouche étant une cartouche hydraulique, comprenant en outre :
un implant (120) disposé dans le compartiment d'implants (110) ;
un boîtier (125) couplé au compartiment d'implants (110) ;
dans lequel :
le piston (135) est configuré pour coulisser dans le boîtier (125) ; l'alésage (140) à travers le piston (135) étant couplé fluidiquement au compartiment d'implant (110) ;
la tige de piston (130) ayant une extrémité filetée disposée à l'intérieur du boîtier (125) ; et
comprenant en outre :
un écrou de guidage (150) enfilé sur l'extrémité filetée de la tige de piston (130) ; et
la chambre (165) étant configurée pour recevoir la cartouche hydraulique (175) entre le piston (135) et l'écrou de guidage (150) ;
la tige de piston (130) étant configurée pour être poussée de manière à déplacer rigidement l'écrou de guidage (150), la cartouche hydraulique (175), et le piston (130) afin de faire avancer l'implant (120) du compartiment d'implant (110) à la buse (105), et la tige de piston (130) étant configurée pour tourner à travers l'écrou de guidage (150) afin d'entraîner un fluide de travail (180) de la cartouche hydraulique (175) à travers l'alésage (140) pour faire avancer l'implant (120) à travers la buse (105).

12. Système selon la revendication 7 ou système selon la revendication 11, le piston (135) comprenant un accouplement (145) configuré pour recevoir la cartouche hydraulique (175) et pour coupler de manière fluidique la cartouche hydraulique (175) à l'alésage (140).

13. Système selon la revendication 11 ou la revendication 12, l'écrou de guidage (150) étant configuré pour maintenir une position relative de la tige de piston (130) et de la cartouche hydraulique (175) lorsque l'implant (120) est avancé du compartiment d'implant (110) à la buse (105).

14. Système selon l'une quelconque des revendications 11 à 13,
la cartouche hydraulique (175) comprenant un joint d'étanchéité de cartouche (190) ; et
la tige de piston (130) étant configurée pour faire avancer le joint d'étanchéité de cartouche (190) afin d'entraîner le fluide de travail de la cartouche hydraulique (175) à travers l'alésage (140).

15. Système selon la revendication 1, comprenant en outre :
un implant (120) disposé dans le compartiment d'implants (110) ;
un boîtier (125) couplé au compartiment d'implants (110) ;
dans lequel :
le piston (135) est configuré pour coulisser à l'intérieur du boîtier (125) ;
l'alésage (140) à travers le piston (135) est couplé fluidiquement au compartiment d'implant (110) ;
la tige de piston (130) ayant une extrémité filetée disposée à l'intérieur du boîtier (125) ;
comprenant en outre :
un écrou de guidage (150) enfilé sur l'extrémité filetée de la tige de piston (130),
la chambre (165) étant entre le piston (135) et l'écrou de guidage (150) ; et
la cartouche (175) étant disposée dans la chambre (165), la cartouche (175) comportant un embout (185) couplé fluidiquement à l'alésage (140) à travers le piston (135), un joint d'étanchéité de cartouche (190), et un fluide de travail disposé entre l'embout (185) et le joint d'étanchéité de cartouche (190) ;
la tige de piston (130) étant configurée pour être poussée pour faire avancer l'écrou de guidage (150), la cartouche (175), le piston (135) et l'implant (120) du compartiment d'implant (110) à la buse (105), et la tige de piston (135) étant configurée pour tourner à travers l'écrou de guidage (150) pour faire avancer le joint d'étanchéité de cartouche (190), entraînant ainsi le fluide de travail (180) à travers l'alésage (140) pour faire avancer l'implant (120) à travers la buse (105).

16. Système d'administration d'un implant à l'aide d'une cartouche hydraulique pour chirurgie oculaire, le système comprenant :
une buse (105) ;
un compartiment d'implant (110) accouplé à la buse (105) ;
un implant (120) disposé dans le compartiment d'implants (110) ;
un boîtier (125) couplé au compartiment d'implants (110) ;
un piston (135) disposé à l'intérieur du boîtier (125) ;
un alésage (140) à travers le piston (135), l'alésage (140) étant couplé fluidiquement au compartiment d'implant (110) ;
une tige de piston (135) disposée au moins partiellement à l'intérieur du boîtier (125) ; et
une chambre (165) configurée pour recevoir la cartouche hydraulique (175) entre le piston (135) et la tige de piston (130) ;
la chambre (165) étant configurée pour faire avancer l'implant (120) du compartiment d'implant (110) à la buse (105) dans une première phase d'administration, et
la tige de piston (130) étant actionnable pour entraîner un fluide de travail (180) de la cartouche hydraulique (175) à travers l'alésage (140) pour faire avancer l'implant (120) à travers la buse (105) dans une seconde phase d'administration.
